# EUROPÄISCHE PATENTANMELDUNG

(11) **EP 1 059 094 A1**
(43) Veröffentlichungstag der Anmeldung: **13.12.2000**
(21) Anmeldenummer: 99111325.9
(22) Anmeldetag: 10.06.1999
(51) Int. Cl.: A61M 1/00

(54) **Blutsammelvorrichtung**

(71) Anmelder: Fresenius AG, 61352 Bad Homburg v.d.H. (DE)
(72) Erfinder: Biesel, Wolfgang Dr., 66564 Ottweiler (DE); Witthaus Friedrich, D-66640 Namborn (DE)
(74) Vertreter: Laufhütte, Dieter, Dr.-Ing.

(57) **Zusammenfassung**

Die Erfindung betrifft eine Blutsammelvorrichtung mit einem Unterdruckbehältnis und einem dieses verschließenden Deckel. An dem Deckel befindet sich ein Blutfilter. Erfindungsgemäß weist der Blutfilter einen flexibel ausgestalteten bzw. beweglich gehaltenen Filterboden auf.

## Beschreibung

Die Erfindung betrifft eine Blutsammelvorrichtung nach dem Oberbegriff des Anspruchs 1.

Bei Operationen fällt häufig an der Operationsstelle sehr viel Blut an, das früher häufig nur abgesaugt und dann anschließend verworfen wurde. Da dieses Blut aber im Hinblick auf die Versorgung des Patienten mit Spenderblut eine sehr wertvolle Bereicherung und Ergänzung sein kann, wurde bereits mehrfach vorgeschlagen, das Blut abzusaugen und wieder aufzuarbeiten, so daß es dem Patienten wieder zugeführt werden kann.

So ist beispielsweise aus der EP 0 669 139 A bereits eine gattungsgemäße Blutsammelvorrichtung vorbekannt, in welcher ein Unterdruck erzeugt wird, um von einer Operationsstelle Blutgemisch anzusaugen. Dieses Blut ist in der Regel verunreinigt mit Blutgerinnseln, Gewebeteilchen, Gewebefasern und dergleichen und ist mehr oder minder stark verdünnt, und mit Antikoagulanz versetzt. Die partikulären Verunreinigungen werden abfiltriert und das Restblut dem Patienten wieder transfundiert.

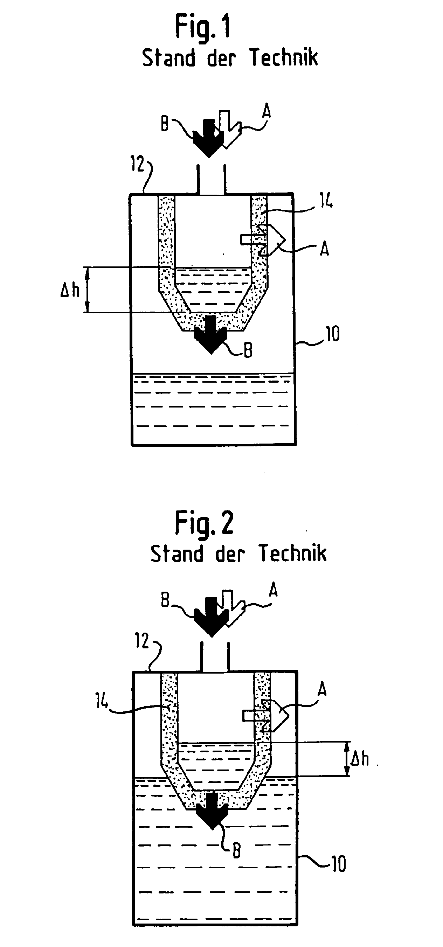

Der Aufbau der bekannten Blutsammelvorrichtung nach der EP 0 669 139 A ist schematisch in den Fig. 1 und 2 wiedergegeben. Das nicht näher dargestellte Unterdruckbehältnis der Blutsammelvorrichtung weist einen inneren Beutel 10 auf, der fest mit einem Deckel 12 verbunden ist. Ebenfalls mit dem Deckel 12 ist ein starres Filtersieb 14 verbunden. Das Filtersieb 14 ragt in den Beutel 10, wie in den Fig. 1 und 2 gezeigt, hinein. Zwischen dem Beutel 10 und dem hier nicht näher dargestellten Unterdruckbehältnis wird Unterdruck gezogen, wodurch sich der Beutel ausdehnt und das Blut ansaugt. Zwischen dem Beutel 10 und dem Filter 14 ist in hier nicht näher dargestellter Art und Weise ein Ableitschlauch für das filtrierte Blutgemisch angeordnet.

Nachteilig bei der vorbekannten Blutsammelvorrichtung ist es, daß der Filter in den Beutel hineinragt. Aufgrund der beutelartigen Konstruktion des Filters trennt sich der Flüssigkeitsstrom vom Luftstrom beim Eintritt in die Blutsammelvorrichtung und wird auf getrennten Wegen durch den Filter geführt, wie das durch die Pfeile A für die Luft und B für das Blutgemisch in den Fig. 1 und 2 dargestellt ist.

Das Blut tropft in den Filterbeutel 14, wohingegen der Luftstrom den Filter an der Seite passiert. Die Druckdifferenz, die das Blut durch den Filter treibt, entsteht durch den Füllstand des Blutes im Filterbeutel 14, d.h. durch den hydrostatischen Druck der Flüssigkeitssäule auf dem Filter. Bei eingetauchtem Filterbeutel entsteht der Druck durch den geodätischen Höhenunterschied zwischen dem Füllstand innerhalb und außerhalb des Filters, wie dies in Fig. 2 dargestellt ist. Die treibende Druckdifferenz liegt typischerweise bei maximal 0,015 bar, d.h. bei ca. 15 cm Flüssigkeitssäule. Der Filterbeutel läuft somit nur langsam leer und leert sich bei Benetzung des Filters z.B. mit Gerinnseln gar nicht mehr. Das Blut bleibt während einer langen Aufenthaltszeit im Filterbeutel mit gerinnungsaktivierenden festeren Abfallbestandteilen, wie beispielsweise Gewebefetzen in Kontakt. Dies kann durch zusätzliche Gerinnselbildung zur Blockade des Filters führen. Das Blut wird vom Filter zurückgehalten und kann nicht mehr aus dem Reservoir entnommen werden.

Es sind weitere Blutsammelgefäße bekannt, die keinen inneren Beutel aufweisen. Diese starren Gefäße weisen an ihrer Oberseite einen Blutgemischeingang und einen Luftabzug auf. Der Blutausgang hingegen kann im Deckel oder am Boden sein. Befindet er sich im Deckel, führt ein Ableitschlauch vom Boden bis zum Dekkel. Befindet sich der Ausgang am Boden, läuft das Blutgemisch über Schwerkraft aus dem Behälter.

Bevorzugt ist jedoch eine Ausführungsform mit innerem flexiblen Beutel, da lediglich der innere flexible Beutel nach beendeter Blutsammlung ersetzt wird, wodurch das Disposable-Teil preisgünstig wird.

Aufgabe der Erfindung ist es, eine gattungsgemäße Blutsammelvorrichtung weiterzubilden, um ein Zusetzen und Verstopfen des Filters zu verhindern und andererseits die Zellwiedergewinnung der Blutzellen zu erhöhen.

Erfindungsgemäß wird ausgehend von einer gattungsgemäßen Blutsammelvorrichtung diese Aufgabe durch die zusätzlichen kennzeichnenden Merkmale des Anspruchs 1 gelöst.

Der Blutfilter ist im Deckel oder unmittelbar an diesen anschließend in Form einer Filterkammer ausgebildet. Erfindungsgemäß ist nur der Boden der Filterkammer als Filter ausgebildet, wobei dieser Filter an den Rändern eingefaßt und nicht unterstützt wird. Hierdurch hält die Filterfläche eine Flexibilität in Strömungsrichtung. Überraschenderweise wird durch diese erhöhte Flexibilität eine Verminderung des Flusses oder ein Verschluß vollständig vermieden, was durch die Verschiebung und Verlagerung des zurückgehaltenen Gruppenmaterials aufgrund der Bewegung des Filters erklärt werden kann.

Zusätzlich kann dadurch, daß der Filter nicht mehr als Beutel ausgebildet ist, der in das Blutreservoir hineinragt, zunächst verhindert werden, daß der Luftstrom das zu filternde Blut auf getrennte Wege durch den Filter führt. Der Blutfilter ist nun derart ausgestaltet, daß der Luftstrom parallel zum Flüssigkeitsstrom durch die Filterfläche des Blutfilters geführt wird. Damit ist der durch den Unterdruck erzeugte Luftstrom die treibende Kraft für die Filtration. Die maximale Druckdifferenz, die bei dieser erfindungsgemäßen Blutsammelvorrichtung das Blut durch den Filter treibt, liegt in der Höhe des Vakuumdrucks von ca. 0,1 bar. Dies führt dazu, daß innerhalb der Filteranordnung das Blut nur eine sehr kurze Aufenthaltszeit hat und sehr schnell durch den Filter geleitet wird. Die Filterleistung ist erhöht und ermöglicht eine Reduktion der Filtergröße und aufwendiger Behandlungsschritte für das Filtermedium, wie beispielsweise eine Silikonisierung zur Erhöhung der Filterleistung. Andererseits wird die Kontaktzeit zu gerinnungsaktiven festen Abfallbestandteilen auf ein Minimum reduziert. Aufgrund der Integration des Filters in den Deckel taucht diese auch nicht in das zwischengelagerte Blut ein. Der Filter hält praktisch kein Blut zurück, wodurch die Zellwiedergewinnung der Blutzellen im Filter entscheidend erhöht wird.

Weitere Einzelheiten und Vorteile der Erfindung werden anhand eines in der Zeichnung dargestellten Ausführungsbeispiels näher erläutert. Es zeigen:
- Fig. 1 und 2:: Schematisch die Blutsammelvorrichtung nach dem Stand der Technik, die zuvor beschrieben wurde,
- Fig. 3:: eine schematische Darstellung einer Ausführungsform der erfindungsgemäßen Blutsammelvorrichtung in einem ersten Füllzustand und
- Fig. 4:: die Blutsammelvorrichtung gemäß Fig. 3 in einem zweiten Füllzustand.

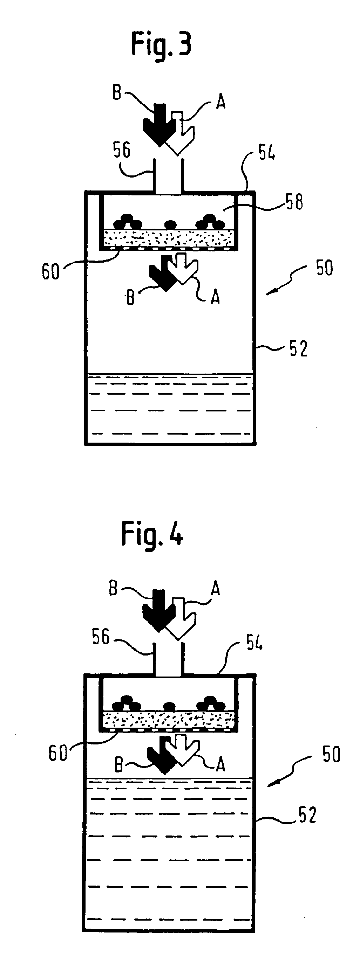

Die in den Fig. 3 und 4 dargestellte Blutsammelvorrichtung zeigt nur eine schematische Darstellung. Die grundsätzliche Funktion der hier näher beschriebenen Blutsammelvorrichtung entspricht derjenigen gemäß der EP 0 669 139 A als bevorzugte Ausführung. In den Fig. 3 und 4 ist aus Gründen der Vereinfachung bei der Blutsammelvorrichtung 50 zunächst lediglich der Beutel 52 dargestellt, der an einem Deckel 54 anschließt. Dies kann auch ein starrer Behälter sein. Im Deckel 54 ist ein Anschluß für einen Blutzuführschlauch 56 vorgesehen. Im Deckel 54 ist weiterhin eine Filterkammer 58, deren Boden als ebenes flexibles Filterelement 60 gebildet ist, integriert, während die Seitenwände nur als Halterung ausgebildet sind. Die Unterdruckleitung muß bei Verwendung eines flexiblen Beutels zwischen Beutel und Unterdruckbehältnis angeordnet sein, bei einem starren Behälter im Deckel. Die Unterdruckleitung selbst sowie der Ableitschlauch für das filtrierte Blutgemisch sind aus Vereinfachungsgründen hier nicht näher dargestellt.

Aus der Fig. 3 wird deutlich, daß sowohl die angesaugte Luft A wie auch das angesaugte Blut B in die Filterkammer 58 eingesaugt werden. Sowohl die Luft A wie auch das Blut B werden zusammen durch den flexiblen ebenen Filter 60 gesaugt. Hierdurch füllt sich das Reservoir 52 mit Blut. In Fig. 3 ist das Reservoir noch wenig gefüllt, während er in Fig. 4 in seinem gefüllten Zustand dargestellt ist. Selbst in dem in Fig. 4 dargestellten gefüllten Zustand liegt der in den Deckel 54 integrierte Filter nicht in dem gesammelten Blut.

Mit der erfindungsgemäßen Blutsammelvorrichtung können höhere Differenzdrücke erzeugt werden. Daraus folgend können höhere Flüsse und ein kleineres Restblutvolumen im Filter erreicht werden. Insgesamt kann ein kleinerer Filter Verwendung finden und das Blut hat eine geringere Aufenthaltszeit bzw. Kontaktzeit mit dem Filter.

## Patentansprüche

1. Blutsammelvorrichtung mit einem Unterdruckbehältnis, einem diesen verschließenden Deckel, an den eine Zuleitung für abgesaugtes Blut ansetzt, wobei sich das ... Unterdruckbehältnis an diesen Deckel anschließt und wobei an dem Deckel unmittelbar ein Blutfilter anschließt,
**dadurch gekennzeichnet,**
daß der Filterboden des Blutfilters flexibel ausgestaltet ist bzw. beweglich ist.

2. Blutsammelvorrichtung nach Anspruch 1, dadurch gekennzeichnet, daß der Blutfilter waagerecht ausgebildet ist.

3. Blutsammelvorrichtung nach Anspruch 1 oder 2, dadurch gekennzeichnet, daß im Deckel oder an diesen anschließend eine Filterkammer ausgebildet ist, deren Boden und/oder Seitenwände der Blutfilter ist.

4. Blutsammelvorrichtung nach einem der Ansprüche 1 bis 3, dadurch gekennzeichnet, daß die Filterkammer breiter als hoch ist.

5. Blutsammelvorrichtung nach einem der Ansprüche 1 bis 4, dadurch gekennzeichnet, daß die seitlichen Flächen des Blutfilters Halterungen darstellen, die den Boden, der als Filterfläche ausgebildet ist, halten.
